# EUROPEAN PATENT APPLICATION

(11) **EP 1 666 027 A1**
(43) Date of publication of application: **07.06.2006**
(21) Application number: 04772760.7
(22) Date of filing: 03.09.2004
(51) Int. Cl.: A61K 9/107, A61K 47/44, A61K 47/34, A61K 47/38, A61K 47/24, A61K 31/05, A61K 31/045, G02C 13/00

(54) **OPHTHALMIC COMPOSITION FOR CONTACT LENS**

(30) Priority: 10.09.2003 JP 2003319059; 11.09.2003 JP 2003319148
(71) Applicant: Senju Pharmaceutical Co., Ltd., Osaka-shi, Osaka 541-0046 (JP)
(72) Inventor: NAKAYAMA, Hisayuki, 6220826 (JP); NEMOTO,Fukiko, 12-15-104,Zenkaiminamimachi 2-chome, Hyogo 6512109 (JP)
(74) Representative: von Kreisler, Alek
(86) International application number: PCT/JP2004/012811
(87) International publication number: WO 2005/025539

(57) **Abstract**

Disclosed are; a method for suppressing adsorption of a refreshing agent and/or chlorobutanol by a contact lens in an aqueous ophthalmic composition for contact lens containing a refreshing agent and/or chlorobutanol, as well as for suppressing pH decline due to degradation of chlorobutanol, wherein the method comprises preparing the composition in the form of an oil-in-water type emulsion. An ophthalmic composition for contact lens in the form of an oil-in-water type emulsion containing a refreshing agent and/or chlorobutanol is also disclosed.

## Description

### TECHNICAL FIELD

The present invention relates to an ophthalmic composition for contact lens, wherein the composition is an oil-in-water type emulsion comprising a refreshing agent, an oil and an emulsifying agent contained in water, as well as to a method for suppressing adsorption of a refreshing agent by a contact lens.

The present invention further relates to a method for suppressing adsorption of chlorobutanol contained in an aqueous ophthalmic composition by a contact lens, to a method for suppressing pH decline of an aqueous ophthalmic composition due to degradation of chlorobutanol, and to a ophthalmic composition for contact lens which is an oil-in-water type emulsion containing chlorobutanol.

### BACKGROUND ART

While wearing a contact lens, dryness of the eye is often felt due to the tear film made unstable and increased evaporation of the tear. In the case of a soft contact lens, further, evaporation of water from the contact lens itself leads to decreased water content of the contact lens, thus aggravating the feeling of dryness. In addition, while wearing a contact lens, the wobbling of the contact lens and its edge that are sensed and distortion of the lens, in the case of a soft contact lens, due to decreased water content serve to aggravate foreign body sensation. To soften these symptoms, ophthalmic compositions for contact lens have been used. Reflecting increased diversities in the tastes of contact lens users as well as the increased number of contact lens users in recent years, refreshing agent-containing ophthalmic compositions for contact lens have come to be launched on the market. However, refreshing agents are easily adsorbed by a contact lens, especially a soft contact lens, and, depending on their chemistry or concentration, pose risks of causing pigmentation or deformation of the lens or disorders of the eye through their accumulation in the lens. To solve this problem the applicant has found a method for suppressing adsorption of terpenoids, which are used as refreshing agents, by a soft contact lens by addition of a polyoxyethylenesorbitan ester or by maintaining the pH not lower than 5.5 (see Patent Document 1). Another method has also been proposed in which adsorption of refreshing agents by a soft contact lens is suppressed by addition of polyols (see Patent Document 2). However, a refreshing agent-containing ophthalmic composition for contact lens is still needed which enables further reduction of adsorption of refreshing agents by a contact lens. On the other hand, it is known that employment of an oil-in-water type emulsion serves to suppress adsorption of terpenoids such as menthol and borneol by plastic containers made of, for example, polyolefins such as polypropylene, saturated polyesters such as polyethylene terephthalate, or polycarbonate (see Patent Document 3). However, nothing is known about suppression of their adsorption by a contact lens.

Chlorobutanol, which has topical anesthetic effect, is used in ophthalmic preparations such as eye drops. But it has been a problem that chlorobutanol is easily adsorbed by a contact lens. In addition, it has been another problem that chlorobutanol in an aqueous preparation is susceptible to degradation, esp. at a neutral range, and, when degraded, it lowers the pH of the aqueous preparation, which could be a factor inducing eye irritation in the case of ophthalmic preparations.

Further, either ophthalmic compositions for soft contact lens comprising above refreshing agents together with a polyoxyethylenesorbitan ester and having pH adjusted to 5.5 or higher (Patent Document 1) or the ophthalmic compositions for soft contact lens containing refreshing agents together with polyols (Patent Document 2) are unsatisfactory, since they give insufficient refreshing feeling when the content of refreshing agents is relatively low, whereas they give more irritation than refreshing feeling when the content of refreshing agents is increased.
Patent Document 1: Japanese Patent No. 3090125
Patent Document 2: Japanese Patent Application Publication No. 2001-122774
Patent Document 3: Japanese Patent Application Publication No. 2000-273061

### DISCLOSURE OF INVENTION

### The Problem to be solved by the Invention

Against the above background, an objective of the present invention is to provide a method for suppressing adsorption of refreshing agents by a contact lens, esp., a soft contact lens, and a refreshing agent-containing ophthalmic composition for contact lens (eye drops, eye washes, contact lens wetting liquid) in which adsorption of refreshing agents is minimized by utilizing that method.

Even when high concentrations of a refreshing agent is contained in an ophthalmic composition, if chlorobutanol is contained in an sufficient amount, irritating sensation felt immediately after instillation in the eye can be soften while maintaining the refreshing feeling. However, chlorobutanol is easily adsorbed by a contact lens. Thus, a high amount of chlorobutanol in an ophthalmic composition for contact lens would not only lead to adsorption of increased amount of chlorobutanol by a contact lens (and thereby present a risk of affecting the physical property of the a contact lens), but also could cause disorders the eye due to excessively decline of the pH of the composition during storage, for increased amount of degradation would occur in a composition containing a high concentration of chlorobutanol. For these reasons, it has not been possible to add a sufficient amount of chlorobutanol to ophthalmic compositions for contact lens. Under the circumstances, another objective of the present invention is to provide means to suppress degradation of chlorobutanol in aqueous ophthalmic compositions for contact lens, thereby minimizing pH decline of such compositions, and also suppressing adsorption of chlorobutanol by a contact lens.

Still another objective of the present invention is to provide a chlorobutanol-containing ophthalmic composition for contact lens, wherein chlorobutanol is contained in such a form which allows to keep its stability and suppress its adsorption by a contact lens, as well as an ophthalmic composition for contact lens, which contains chlorobutanol in such a form and further contains a refreshing agent.

### The Means to Solve the Problem

The present inventors conducted a study of refreshing agent-containing ophthalmic compositions for contact lens in search of a preparation form which suppresses adsorption of refreshing agents by a contact lens. As a result, the present inventors found that it is possible to produce an ophthalmic composition for contact lens in which adsorption of refreshing agents by a contact lens is minimized, by adding, along with the refreshing agents, an oil and a emulsifying agent and forming an oil-in-water emulsion.

Further, the present inventors also conducted a study in search of a method to suppress adsorption of chlorobutanol by a contact lens in aqueous preparations and a method to suppress degradation of chlorobutanol. As a result, the present inventors found that a composition is obtained in which adsorption of chlorobutanol by a contact lens is minimized and, in addition, pH decline in the neutral range is delayed because of suppressed degradation of chlorobutanol, by adding, together with chlorobutanol, an oil and an emulsifying agent or a phospholipid in water to form an oil-in-water type emulsion.

The present invention has been accomplished on the basis of the above findings and through further studies.

Thus, the present invention provides:
(1) An ophthalmic composition for contact lens, wherein the composition is an oil-in-water type emulsion comprising a refreshing agent and/or chlorobutanol together with an oil and an emulsifying agent in water.
(2) The ophthalmic composition for contact lens of (1) above, wherein the refreshing agent is at least one compound selected from the group consisting of terpenoids, clove perfume, peppermint perfume, eucalyptus perfume, lemon perfume, laurel perfume, lavender perfume, tarragon perfume, cardamon perfume, cedar perfume, grapefruit perfume, orange perfume, ginger perfume, bergamot perfume, coriander perfume, cinnamon perfume, jasmine perfume, rosemary perfume, rose perfume, pine perfume, cardamon perfume, benzoin perfume, geranium perfume, chamomile perfume, marjoram perfume, spearmint perfume, fennel perfume, vanilla flavor, peppermint oil, peppermint water and bergamot oil.
(3) The ophthalmic composition for contact lens of (2) above, wherein the terpenoid is at least one compound selected from the group consisting of menthol, camphor, borneol, geraniol, menthone, cineol and limonene.
(4) The ophthalmic composition for contact lens of one of (1) to (3) above, wherein the oil is an animal/vegetable oil.
(5) The ophthalmic composition for contact lens of (4) above, wherein the vegetable/animal oil is castor oil.
(6) The ophthalmic composition for contact lens of one of (1) to (5) above, wherein the emulsifying agent is at least one compound selected from the group consisting of surfactants, phospholipids and water-soluble macromolecular compounds.
(7) The ophthalmic composition for contact lens of (6) above, wherein the surfactants are nonionic surfactants.
(8) The ophthalmic composition for contact lens of (7) above, wherein the nonionic surfactants are polyoxyethylenesorbitan fatty acid esters.
(9) The ophthalmic composition for contact lens of one of (6) to (8) above, wherein the phospholipid is lecithin.
(10) The ophthalmic composition for contact lens of one of (6) to (9) above, wherein the water-soluble macromolecular compound is hydroxypropylmethylcellulose.
(11) The ophthalmic composition for contact lens of one of (1) to (10) above, wherein the contact lens is a soft contact lens.
(12) An ophthalmic composition for contact lens, wherein the composition is an oil-in-water type emulsion containing chlorobutanol and a phospholipid in water.
(13) The ophthalmic composition for contact lens of (12) above, wherein the phospholipid is lecithin.
(14) The ophthalmic composition for contact lens of (12) or (13) above further comprising a refreshing agent.
(15) The ophthalmic composition for contact lens of (14) above, wherein the refreshing agent is at least one compound selected from the group consisting of terpenoids, clove perfume, peppermint perfume, eucalyptus perfume, lemon perfume, laurel perfume, lavender perfume, tarragon perfume, cardamon perfume, cedar perfume, grapefruit perfume, orange perfume, ginger perfume, bergamot perfume, coriander perfume, cinnamon perfume, jasmine perfume, rosemary perfume, rose perfume, pine perfume, cardamon perfume, benzoin perfume, geranium perfume, chamomile perfume, marjoram perfume, spearmint perfume, fennel perfume, vanilla flavor, peppermint oil, peppermint water and bergamot oil.
(16) A method for suppressing adsorption of a refreshing agent and/or chlorobutanol by a contact lens in an aqueous ophthalmic composition for contact lens containing the refreshing agent and/or chlorobutanol, wherein the method comprises preparing the composition in the form of an oil-in-water type emulsion by adding an oil and an emulsifying agent.
(17) The method for suppressing adsorption of (16) above, wherein the oil is an animal/vegetable oil.
(18) The method for suppressing adsorption of (17) above, wherein the animal/vegetable oil is castor oil.
(19) The method for suppressing adsorption of one of (16) to (18) above, wherein the emulsifying agent is at least one compound selected from the group consisting of surfactants, phospholipids and water-soluble macromolecular compounds.
(20) The method for suppressing adsorption of (19) above, wherein the surfactants are nonionic surfactants.
(21) The method for suppressing adsorption of (20) above, wherein the nonionic surfactants are polyoxyethylenesorbitan fatty acid esters.
(22) The method for suppressing adsorption of one of (19) to (21) above, wherein the phospholipid is lecithin.
(23) The method for suppressing adsorption of one of (19) to (22) above, wherein the water-soluble macromolecular compound is hydroxypropylmethylcellulose.
(24) A method for suppressing adsorption of chlorobutanol by a contact lens in a chlorobutanol-containing aqueous ophthalmic composition for contact lens, wherein the method comprises preparing the composition in the form of an oil-in-water type emulsion by adding a phospholipid.
(25) The method for suppressing adsorption of (24) above, wherein the phospholipid is lecithin.
(26) A method for suppressing pH decline of a chlorobutanol-containing aqueous ophthalmic composition for contact lens, wherein the method comprises preparing the composition in the form of oil-in-water type emulsion by adding an oil and an emulsifying agent.
(27) The method for suppressing pH decline of (26) above, wherein the oil is an animal/vegetable oil.
(28) The method for suppressing pH decline of (27) above, wherein the animal/vegetable oil is castor oil.
(29) The method for suppressing pH decline of one of (26) to (28) above, wherein the emulsifying agent is at least one compound selected from the group consisting of surfactants, phospholipids and water-soluble macromolecular compounds.
(30) The method for suppressing pH decline of (29) above, wherein the surfactants are nonionic surfactants.
(31) The method for suppressing pH decline of (30) above, wherein the nonionic surfactants are polyoxyethylenesorbitan fatty acid esters.
(32) The method for suppressing pH decline of one of (26) to (31) above, wherein the phospholipid is lecithin.
(33) The method for suppressing pH decline of one of (29) to (32) above, wherein the water-soluble macromolecular compound is hydroxypropylmethylcellulose.
(34) A method for suppressing pH decline of a chlorobutanol-containing aqueous ophthalmic composition for contact lens, wherein the method comprises preparing the composition in the form of oil-in-water type emulsion by adding a phospholipid.
(35) The method for suppressing pH decline of (34) above, wherein the phospholipid is lecithin.

### [The Effect of the Invention]

According to the present invention, adsorption of a refreshing agent and/or chlorobutanol by a contact lens is markedly suppressed. Therefore, it enables to produce ophthalmic compositions for contact lens containing refreshing agents in an increased amount than before, while suppressing the risk of inducing eye irritation by a refreshing agent once adsorbed by a contact lens. Decline of pH is also significantly suppressed through suppression of degradation of chlorobutanol. Therefore, it enables addition of chlorobutanol in an increased amount than before, while avoiding risks of affecting the contact lens and eye irritation by chlorobutanol. Further, as this make it possible to soften an irritating sensation of a refreshing agent, which may be felt immediately after instillation in the eye when increased amount of refreshing agent is contained, it further enables to provide ophthalmic compositions for contact lens containing an increased amount of refreshing agent.

### BEST MODE FOR CARRYING OUT THE INVENTION

The present invention will be described in further detail below.
The ophthalmic composition for contact lens of the present invention is prepared in the form of an oil-in-water type emulsion by adding a refreshing agent and/or chlorobutanol together with an oil and an emulsifying agent or a phospholipid to water. Employment of such a preparation form enables marked suppression of adsorption of a refreshing agent and/or chlorobutanol by a contact lens.

Examples of refreshing agents that may be used in the ophthalmic composition for contact lens of the present invention include terpenoids, clove perfume, peppermint perfume, eucalyptus perfume, lemon perfume, laurel perfume, lavender perfume, tarragon perfume, cardamon perfume, cedar perfume, grapefruit perfume, orange perfume, ginger perfume, bergamot perfume, coriander perfume, cinnamon perfume, jasmine perfume, rosemary perfume, rose perfume, pine perfume, cardamon perfume, benzoin perfume, geranium perfume, chamomile perfume, marjoram perfume, spearmint perfume, fennel perfume, vanilla flavor, peppermint oil, peppermint water and bergamot oil. Examples of terpenoids include, e.g., menthol, menthone, camphor, borneol, geraniol, cineol, limonene, eugenol, citral, pinene, linalol, fenchyl alcohol, ionone, safranal, terpinene and the like. Among these, menthol, camphor, borneol, geraniol, menthone, cineol and limonene are particularly preferred. Any single one of these refreshing agents, or two or more of them in combination, may be employed as desired.

As mentioned above, the ophthalmic composition for contact lens of the present invention markedly suppresses adsorption of a refreshing agent by a contact lens, and it thereby prevents eye irritation that could be caused by an accumulated refreshing agent in the contact lens. Therefore, it enables to increase the concentration of a refreshing agent to be contained than before, providing an ophthalmic composition for contact lens that gives increased refreshing feeling. Thus, the ophthalmic composition for contact lens of the present invention may contain a refreshing agent, preferably in an amount of 0.0005-0.2 (W/V)%, particularly preferably 0.005-0.05 (W/V)%, regardless of whether chlorobutanol is concomitantly contained.

When chlorobutanol is contained in the ophthalmic composition for contact lens of the present invention, its amount is preferably 0.001-2.0 (W/V)%, more preferably 0.01-0.5 (W/V)%, regardless of whether a refreshing agent is concomitantly contained.

In an ophthalmic composition for contact lens of the present invention containing both a refreshing agent and chlorobutanol, irritating sensation by the refreshing agent felt immediately after instillation in the eye is softened by chlorobutanol, which exerts a topical anesthetic effect. Therefore, it is possible to provide an ophthalmic composition for contact lens that can, while avoiding irritating sensation, provides higher refreshing feeling by employing increased concentrations of refreshing agent compared with conventional compositions. Thus, the ophthalmic composition for contact lens of the present invention may contain a refreshing agent preferably in an amount of 0.0005-0.2 (W/V)%, particularly preferably 0.005-0.05 (W/V)%, while avoiding irritating sensation immediately after instillation in the eye.

In the present invention, a "oil" does not mean a essential oil. Apart from this, there is no specific limitation as to which oils may be employed in the present invention. However, preferred are animal/vegetable oils primarily consisting of fatty acid glycerides, and vegetable oils are particularly preferred. Examples of animal/vegetable oils preferably include triglycerides of medium chain fatty acids consisting of 8-24 carbon atoms such as palmitic acid, stearic acid, oleic acid, ricinolic acid, linoleic acid, linolenic acid, behenic acid, lignoceric acid, icosanoic acid, myristic acid, palmitoleic acid, e.g., castor oil, soybean oil, peanut oil, cottonseed oil, olive oil, sesame oil, camellia oil, rapeseed oil, corn oil and Migriol (brand name), and, most preferably, triglycerides of medium chain fatty acid consisting of 14-24 carbon atoms. From the view point that safety has been established, castor oil is more preferred. Any single one of these oils, or two or more of them in combination, may be employed as desired. The content of oil in the ophthalmic composition for contact lens of the present invention is usually 0.005-20 (W/V)%, and preferably 0.1-5(WfV)%, although it may be determined as desired in accordance with the content of a refreshing agent and chlorobutanol, insofar as an oil-in-water type emulsion is formed.

There is no specific limitation as to which emulsifying agents may be employed in the present invention. They include surfactants, phospholipids and water-soluble macromolecular compounds. Any single one of them, or their mixture, may be employed.

Surfactants may be nonionic surfactants, anionic surfactants, cationic surfactants or amphoteric surfactants. Considering their superior emulsifying effect, nonionic surfactants are particularly preferred.

Examples of nonionic surfactants include polyoxyethylenesorbitan fatty acid esters, polyoxyethylenehydrogenated castor oil, polyethyleneglycol fatty acid esters (e.g., polyoxyl stearate), polyoxyethylenepolyoxypropylene alkyl ethers, polyoxyalkylene alkyl phenyl ethers, polyglycerol fatty acids esters (e.g., decaglyceryl monolaureate), glycerol fatty acid esters, sorbitan fatty acid esters, and polyoxyethylenepolyoxypropylene glycol (poloxamer). Particularly preferred are polyoxyethylenesorbitan fatty acid esters, decaglyceryl monolaureate, polyoxyl stearate 40, poloxamer 407 and polyoxyethylenehydrogenated castor oil.

Preferred examples of polyoxyethylenesorbitan fatty acids include polysorbate 80 and polysorbate 20, 40, 60, 65, and 85.

Examples of anionic surfactants include sodium lauroyl sarcosinate, lauroyl-L-glutamic acid triethanolamine, sodium myristyl sarcosinate and sodium lauryl sulfate.

Examples of cationic surfactants include benzethonium chloride, benzalkonium chloride and cetylpyridinium chloride.

Examples of amphoteric surfactants include lauryldimethylaminoacetic acid betaine, 2-alkyl-N-carboxymethyl-N-hydroxyethylimidazolium betaine and alkyldiaminoglycine hydrochloride.

Example of preferred phospholipids include glycerophospholipids, inter alia, lecithin, esp., egg yolk lecithin and soybean lecithin.

Examples of water-soluble macromolecular compounds include methylcellulose, hydroxyethylcellulose, hydroxypropylmethylcellulose, polyvinylalcohol, polyvinylpyrrolidone, cyclodextrin, sodium chondroitin sulfate, carboxymethylcellulose, hyaluronic acid, sodium hyaluronate. Hydroxypropylmethylcellulose, methylcellulose, hydroxyethylcellulose and sodium chondroitin sulfate are preferred, and hydroxypropylmethylcellulose is the most preferred. Any single one of these water-soluble macromolecular compounds, or two or more in combination, may be employed as desired.

The amount of one or more emulsifying agent contained in the ophthalmic composition for contact lens of the present invention may be determined according to the content of a refreshing agent, chlorobutanol and an oil as desired insofar as an oil-in-water type emulsion is formed. It is usually 0.001-20 (W/V)%, preferably 0.1-5 (W/V)%.

In the present invention, a phospholipid may be added to emulsify an oil as described above. However, a phospholipid may be employed as a material that by itself forms an emulsion in water, without help other materials like an oil or an emulsifying agent. In such a case, the content of a phospholipid is usually 0.005-20 (W/V)%, preferably 0.1-5 (W/V)%, insofar as an oil-in-water emulsion is formed.

A variety of pharmacologically active components conventionally employed in eye drops may be added to the ophthalmic composition for contact lens of the present invention containing a refreshing agent and/or chlorobutanol. Examples of such components include antihistamic agents such as chlorpheniramine maleate, antiinflammatories such as dipotassium glycyrrhizinate and ε-aminocaproic acid, vitamins such as pyridoxine hydrochloride, d-α-tocopherol acetate, panthenol and calcium pantothenate, vasoconstrictors such as naphazoline hydrochloride and tetrahydrozoline hydrochloride, as well as aminoethylsulfonic acid, sodium chondroitin sulfate and allantoin.

Furthermore, a variety of additives may be added to the ophthalmic composition for contact lens of the present invention, such as buffers, isotonizers, preservatives, solubilizing agents, stabilizers, chelating agents, thickeners and pH adjusting agents.

Examples of buffers include boric acid or salts thereof (such as borate), citric acid or salts thereof (sodium citrate), tartaric acid or salts thereof (such as sodium tartrate), gluconic acid or salts thereof (sodium gluconate), acetic acid or salts thereof (such as sodium acetate), phosphoric acid or salts thereof (sodium monohydrogen phosphate, sodium dihydrogen phosphate), a variety of amino acids and combinations thereof.

Examples of isotonizers include, e.g., sorbitol, glucose, mannitol, glycerol, propylene glycol, sodium chloride and potassium chloride.

Examples of preservatives include p-hydroxy benzoate esters, benzalkonium chloride, benzethonium chloride, benzyl alcohol, sorbic acid or salts thereof, chlorhexidine gluconate, sodium dehydroacetate, cetylpyridinium chloride, alkyldiaminoethylglycine hydrochloride.

Examples of solubilizing agents include polyvinylpyrrolidone, polyethylene glycol, propylene glycol, polyoxyethylenehydrogenated castor oil 60 and polyoxyl stearate 40.

Examples of stabilizers include ascorbic acid, sodium edetate, cyclodextrins, condensed phosphoric acid or salts thereof, sulfite salts and citric acid or salts thereof.

Examples of chelating agents include sodium edetate, sodium citrate, condensed phosphoric acid or salts thereof (sodium salt of condensed phosphoric acid).

Examples of thickening agents include methylcellulose, hydroxyethylcellulose, hydroxypropylcellulose, hydroxypropylmethylcellulose, sodium chondroitin sulfate, sodium carboxymethylcellulose, polyvinylpyrrolidone, polyvinylalcohol, polyethylene glycol.

Examples of pH adjusting agents include sodium hydroxide, potassium hydroxide, sodium carbonate, sodium bicarbonate, boric acid or salts thereof (borax), hydrochloric acid, citric acid or salts thereof (e.g., sodium citrate, sodium dihydrogen citrate), phosphoric acid or salts thereof (e.g., sodium hydrogen phosphate, potassium dihydrogen phosphate), acetic acid and salts thereof (e.g., sodium acetate, ammonium acetate), tartaric acid and salts thereof (e.g., sodium tartrate).

The pH of the ophthalmic composition for contact lens of the present invention is adjusted to 3-10, preferably 4-9.

The ophthalmic composition for contact lens of the present invention may be provided in the form of an oil-in-water type emulsion by a conventional method. A few non-limiting example of a preferred method are as follows. Briefly, the composition may be prepared by first adding to water an emulsifying agent and, as needed, one or more of the above-mentioned additives, then further adding an oil in which a refreshing agent and/or chlorobutanol is dissolved, forming an emulsion, and adjusting the pH to 3-10 with a pH adjusting agent. Where a phospholipid is employed alone in place of the combination of an oil and an emulsifying agent, the phospholipid is first dispersed in water and the mixture is warmed. To this, with stirring, chlorobutanol is added and, as needed, a refreshing agent, and an emulsion if formed. After cooling to room temperature, one or more of the above-mentioned additives are added as needed, and the pH then is adjusted to 3-10 with a pH adjusting agent. For providing a homogeneous emulsion, conventional means may be applied, e.g., a mixer, a homogenizer, a homo-mixer, a microfluidizer and a high pressure homogenizer.

As it markedly suppresses adsorption of a refreshing agent and chlorobutanol by a contact lens, the ophthalmic composition for contact lens of the present invention can be applied while wearing one of a variety of contact lenses, such as a non-oxygen permeable hard contact lens, an oxygen permeable hard contact lens and a soft contact lens, and most advantageously while wearing, among them, a soft contact lens.

The ophthalmic composition for contact lens of the present invention may be used as eye drops, an eye wash and a wetting liquid, for contact lens.

### Example 1

The present invention will be described below in further detail with reference to test examples and working examples. However, it should be noted that they are nothing more than examples and do not limit the present invention.

[Test Example 1]
(Test Method)
Test liquids shown in Table 1 below were prepared and used for testing adsorption of 1-menthol by contact lenses.

[Table 1]

**Table 1 Formulas (in 100 ml)**

| | Test liquid 1 (solution) | Test liquid 2 (solution) | Test liquid 3 (emulsion) |
|---|---|---|---|
| 1-Menthol | 0.002 g | 0.002 g | 0.002 g |
| Polysorbate 80 | - | 1.0 g | 1.0 g |
| Castor oil | - | - | 1.0 g |
| Sodium chloride | 0.9 g | 0.9 g | 0.9 g |
| Hydrochloric acid | q.s. | q.s. | q.s. |
| Sodium hydroxide | q.s. | q.s. | q.s. |
| Purified water | q.s. | q.s. | q.s. |
| pH | 7.0 | 7.0 | 7.0 |

Two ml each of the above test liquids were taken in a vial and a contact lens was immersed in it. After 2-hours immersion at 25 °C, the contact lens was removed and the remaining liquid was measured for 1-menthol content by gas chromatography under the following conditions. Those liquid treated likewise but without immersing a contact lens in them were served as control liquids. A residual rate of 1-menthol was calculated as a proportion (%) of 1-menthol content of each test liquid to that of the control liquid, and adsorbed amount of 1-menthol (µg/lens) as the difference between those contents. The test was carried out three times for each test solution and their mean value was obtained. Contact lenses employed were 1-Day Acuview (Johnson & Johnson K.K.)(soft contact lens, material: polymer from HEMA and MAA).

Gas Chromatography Conditions
Detector: Hydrogen flame ionization detector
Column: PEG20M (3 mm x 2 mm)
Column Temp: 160 °C
Carrier Gas: Nitrogen
Detector Temp: 200 °C
Inlet Temp. 200 °C
Range: 1
ATTEN: -6
Flow: Adjusted so that the retention time for 1-menthol falls around 9 min (about 20 ml/min)

(Test Results)
The results of the test are shown in Table 2.

[Table 2]

**Table 2**

| | Measurement No. | Residual rate (%) | Adsorbed amount (µg/lens) | Mean adsorbed amount (µg/lens) |
|---|---|---|---|---|
| Test liquid 1 (solution) | 1 | 87.5 | 4.14 | 3.97 |
| | 2 | 87.8 | 4.08 | |
| | 3 | 88.9 | 3.68 | |
| Test liquid 2 (solution) | 1 | 89.8 | 3.50 | 2.22 |
| | 2 | 95.7 | 1.38 | |
| | 3 | 94.6 | 1.78 | |
| Test liquid 3 (emulsion) | 1 | 97.7 | 0.70 | 1.05 |
| | 2 | 95.1 | 1.56 | |
| | 3 | 97.2 | 0.90 | |

As evident from the results, it is noted that adsorption of 1-menthol by contact lenses is markedly suppressed by employing the form of oil-in-water type emulsion containing polysorbate 80 and castor oil together with 1-menthol.

[Test Example 2]
(Test Method)
Test liquids as shown in Table 3 below were prepared and tested for chlorobutanol adsorption by contact lenses.

[Table 3]

**Table 3 Formulas (in 100 ml)**

| | Test liquid 1 (solu- tion) | Test liquid 2 (solution) | Test liquid 3 (emulsion) | Test liquid 4 (emulsion) | Test liquid 5 (emulsion) | Test liquid 6 (emulsion) | Test liquid 7 (emulsion) |
|---|---|---|---|---|---|---|---|
| Chlorobutanol | 0.2 g | 0.2 g | 0.2 g | 0.2 g | 0.2 g | 0.2 g | 0.2 g |
| Castor oil | - | - | 1g | - | 1g | - | 1g |
| Polysorbate 80 | - | 1g | 1 g | - | - | - | - |
| Egg yolk lecithin | - | - | - | 1g | - | - | - |
| HPMC | - | - | - | - | - | 1g | 1 g |
| Boric acid | 0.5g | 0.5g | 0.5g | 0.5g | 0.5g | 0.5g | 0.5g |
| Sodium chloride | 0.65 g | 0.65 g | 0.65 g | 0.65 g | 0.65 g | 0.65 g | 0.65 g |
| Sodium Hydroxide | q.s. | q.s. | q.s. | q.s. | q.s. | q.s. | q.s. |
| Purified water | q.s. | q.s. | q.s. | q.s. | q.s. | q.s. | q.s. |
| pH | 7.0 | 7.0 | 7.0 | 7.0 | 7.0 | 7.0 | 7.0 |

| | | | | | | | |
|---|---|---|---|---|---|---|---|
| HPMC: Hydroxypropylmethylcellulose 2910 (Metolose 60S H4000, Shin-Etsu Chemical Co., Ltd.) | | | | | | | |

Two ml each of the above test liquids were taken in a vial and a contact lens was immersed in it. After 2-hours immersion at 25 °C, the contact lens was removed and the remaining liquid was measured for chlorobutanol content by high performance liquid chromatography under the following conditions. Those liquid treated likewise except for immersing a contact lens in it were served as control liquids. Adsorbed amount of chlorobutanol per lens was calculated as the difference in the content of chlorobutanol between the test liquids and the control liquid. The test was carried out three times for each test solution and their mean adsorption amount was obtained. Using the following calculation formula, and as comparison with the Test liquid 1, which did not contain castor oil, polysorbate 80, egg yolk lecithin or HPMC, mean suppression rate of chlorobutanol adsorption was determined for the other test liquids.

((Mean adsorbed amount for Test liquid 1 - Mean adsorbed amount for each of the other Test liquids)/Mean adsorbed amount for Test liquid 1)) x 100 (%)

Contact lenses employed were Medalist (Bausch & Lomb Japan K.K., Group I soft contact lenses (water content less than 50 %, nonionic)), and 1-Day Acuview (Johnson & Johnson K.K., Group IV soft contact lenses (water content not less than 50 %, ionic), materials; polymer from HEMA and MAA).

High Performance Liquid Chromatography Conditions:
Detector: Differential refractometer detector
Column: YMC A-302
Column Temp : Constant temperature at about 40 °C
Mobile Phase: Mixture solution of water and methanol (1:1)
Flow: Adjusted that the retention time for chlorobutanol falls around 5 min

(Test Results)
Three separate tests were carried out. The results are shown in Tables 4-6. Suppression rate of chlorobutanol adsorption for each test liquid, which is determined from the results of the above tests, is shown in Table 7. (As for Test liquid 6, mean suppression rate was not calculated because mean absorbed amount for the liquid exceeded the amount for Test liquid 1).

[Table 4]

**Table 4**

| | | Adsorbed amount (µg/lens) | | | Mean adsorbed amount (µg/lens) |
|---|---|---|---|---|---|
| Measurement No. | | 1 | 2 | 3 | |
| Test liquid 1 (solution) | Medalist | 302.78 | 284.52 | 301.08 | 296.13 |
| | 1-Day Acuview | 277.22 | 276.46 | 256.60 | 270.09 |
| Test liquid 2 (solution) | Medalist | 237.00 | 194.02 | 240.36 | 223.79 |
| | 1-Day Acuview | 201.66 | 206.36 | 198.84 | 202.29 |
| Test liquid 3 (emulsion) | Medalist | 144.58 | 142.32 | 116.62 | 134.51 |
| | 1-Day Acuview | 112.90 | 106.38 | 93.50 | 104.26 |

[Table 5]

**Table 5**

| | | Adsorbed amount (µg/lens) | | | Mean adsorbed amount (µg/lens) |
|---|---|---|---|---|---|
| Measurement No. | | 1 | 2 | 3 | |
| Test liquid 1 (solution) | Medalist | 245.96 | 261.90 | 226.08 | 244.65 |
| | 1-Day Acuview | 177.24 | 212.64 | 189.44 | 193.11 |
| Test liquid 4 (emulsion) | Medalist | 173.68 | 161.78 | 186.08 | 173.85 |
| | 1-Day Acuview | 128.92 | 124.08 | 137.00 | 130.00 |
| Test liquid 5 (emulsion) | Medalist | 146.42 | 151.12 | 134.00 | 143.85 |
| | 1-Day Acuview | 124.02 | 108.56 | 115.60 | 116.06 |

[Table 6]

**Table 6**

| | | Adsorbed amount (µg/lens) | | | Mean adsorbed amount (µg/lens) |
|---|---|---|---|---|---|
| Measurement No. | | 1 | 2 | 3 | |
| Test liquid 1 (solution) | Medalist | 381.34 | 311.46 | 251.92 | 314.91 |
| | 1-Day Acuview | 291.16 | 273.42 | 296.78 | 287.12 |
| Test liquid 6 (solution) | Medalist | 393.24 | 310.16 | 249.08 | 317.49 |
| | 1-Day Acuview | 384.04 | 347.96 | 307.16 | 346.39 |
| Test liquid 7 (emulsion) | Medalist | 200.98 | 176.92 | 233.16 | 203.96 |
| | 1-Day Acuview | 132.98 | 198.06 | 200.80 | 177.28 |

[Table 7]

**Table 7**

| | Rate of adsorption suppression (%) | | |
|---|---|---|---|
| | Medalist | 1-Day Acuview | Mean rate of adsorption suppression (%) |
| Test liquid 2 (solution) | 24.4 | 25.1 | 24.8 |
| Test liquid 3 (emulsion) | 54.6 | 61.4 | 58.0 |
| Test liquid 4 (emulsion) | 28.9 | 32.7 | 30.8 |
| Test Liquid 5 (emulsion) | 41.2 | 39.9 | 40.6 |
| Test Liquid 7 (emulsion) | 35.2 | 38.3 | 36.8 |

As shown in the test results above, hydroxypropylmethylcellulose (Test liquid 6) had no suppressive effect on adsorption of chlorobutanol by contact lenses. In contrast, polysorbate 80 (Test liquid 2) was found to suppress adsorption of chlorobutanol by contact lenses. In addition, more potent suppressive effect was noted when egg yolk lecithin was employed alone as an emulsion forming material (Test liquid 4), and the most potent suppressive effects were observed when castor oil plus polysorbate 80 (Test liquid 3), castor oil plus egg yolk lecithin (Test liquid 5) or castor oil plus hydroxypropylmethylcellulose (Test liquid 7) were used to form oil-in-water type emulsions. These results indicate that chlorobutanol adsorption by a contact lens is markedly suppressed by making use of the form of an oil-in-water type emulsion containing it.

[Test Example 3]
(Test Method)
Test liquids shown in Table 8 were prepared and, following filter-sterilization, filled in colorless glass ampoules (5 ml volume). After storage under a condition for an accelerated test, i.e., at 40 °C, 75 % (relative humidity), for three months, pH was measured. The amount of chlorobutanol was also determined and its residual rate was calculated.

[Table 8]

**Table 8 Formulas (in 100 ml)**

| | Test liquid 8 (emul- sion) | Test liquid 9 (solu- tion) | Test liquid 10 (emul- sion) | Test liquid 11 (solu- tion) | Test liquid 12 (emul- sion) | Test liquid 13 (solu- tion) | Test liquid 14 (solution) |
|---|---|---|---|---|---|---|---|
| Chlorobutanol | 0.2 g | 0.2 g | 0.2 g | 0.2 g | 0.2 g | 0.2 g | 0.2 g |
| Castor oil | 2 g | - | 5 g | - | 10 g | - | - |
| Polysorbate 80 | 2 g | 2 g | 5 g | 5 g | 10 g | 10 g | - |
| Sodium citrate | 0.2 g | 0.2 g | 0.2 g | 0.2 g | 0.2 g | 0.2 g | 0.2 g |
| Sodium chloride | 0.9 g | 0.9 g | 0.9 g | 0.9 g | 0.9 g | 0.9 g | 0.9 g |
| Hydrochloric acid | q.s. | q.s. | q.s. | q.s. | q.s. | q.s. | q.s. |
| Sodium hydroxide | q.s. | q.s. | q.s. | q.s. | q.s. | q.s. | q.s. |
| Purified water | q.s. | q.s. | q.s. | q.s. | q.s. | q.s. | q.s. |
| pH | 7.0 | 7.0 | 7.0 | 7.0 | 7.0 | 7.0 | 7.0 |

(Test Results)
The results of the test results are shown in Table 9.

[Table 9]

**Table 9 Formulas (in 100 ml)**

| | Test liquid 8 (emul- sion) | Test liquid 9 (solu- tion) | Test liquid 10 (emul- sion) | Test liquid 11 (solu- tion) | Test liquid 12 (emul- sion) | Test liquid 13 (solu- tion) | Test liquid 14 (solution) |
|---|---|---|---|---|---|---|---|
| pH | 5.74 | 5.54 | 5.96 | 5.66 | 6.17 | 5.58 | 5.34 |
| Residual rate of chlorobutanol (%) | 93.8 | 91.3 | 96.8 | 91.4 | 96.7 | 94.5 | 87.7 |

As evident from the accelerated test, it is noted that pH decline of chlorobutanol-containing solution is sufficiently suppressed over a long period of time by preparing it in the form of an oil-in-water type emulsion using castor oil plus polysorbate 80.

Preparation examples of the ophthalmic composition for contact lens according to the present invention will be shown below.

**[Preparation Example 1] Eye drops**

| | |
|---|---|
| Chlorpheniramine maleate | 0.003 g |
| Dipotassium glycyrrhizinate | 0.025 g |
| Pyridoxine hydrochloride | 0.01 g |
| d-α-Tocopherol acetate | 0.005 g |
| Aminoethylsulfonic acid | 0.1 g |
| Boric acid | 1.6 g |
| Sodium edetate | 0.005 g |
| Borax | q.s. |
| Polyoxyethylenehydrogenated castor oil 60 | 0.1 g |
| 1-Menthol | 0.002 g |
| Chlorhexidine gluconate solution (20 W/V%) | 0.025 ml |
| Castor oil | 1 g |
| Purified water | to 100 ml |
| pH | 7.0 |

According to the formula above, water was added to polyoxyethylenehydrogenated castor oil 60 to dissolve, and the mixture was warmed. To this, with vigorous stirring in a homo-mixer, was added castor oil containing dissolved 1-menthol to form an emulsion. After cooling down to room temperature, to this were added chlorpheniramine maleate, dipotassium glycyrrhizinate, pyridoxine hydrochloride, d-α-tocopherol acetate, aminoethylsulfonic acid, boric acid, sodium edetate and the chlorhexidine gluconate solution, and then borax to adjust the pH, and the mixture was made to volume, which was filter-sterilized to give eye drops.

**[Preparation Example 2] Eye drops**

| | |
|---|---|
| Sodium chloride | 0.55 g |
| Potassium chloride | 0.15 g |
| Potassium L-aspartate | 1.0 g |
| Sodium citrate | 0.2 g |
| Boric acid | 0.4 g |
| Polysorbate 80 | 15 g |
| Sodium edetate | 0.01 g |
| Borax | q.s. |
| 1-Menthol | 0.005 g |
| Borneol | 0.001 g |
| Geraniol | 0.001 g |
| Benzalkonium chloride | 0.005 g |
| Castor oil | 12 g |
| Purified water | to 100 ml |
| pH | 6.0 |

According to the formula above, water was added to polysorbate 80 to dissolve, and the mixture was warmed. To this, with vigorous stirring in a homo-mixer, was added castor oil containing dissolved 1-menthol, borneol and geraniol to form an emulsion. After cooling down to room temperature, to this were added sodium chloride, potassium chloride, potassium L-aspartate, boric acid, sodium citrate, sodium edetate and benzalkonium chloride, and then borax to adjust the pH, and the mixture was made to volume, which was filter-sterilized to give eye drops.

**[Preparation Example 3] Eye drops**

| | |
|---|---|
| Sodium chloride | 0.55 g |
| Potassium chloride | 0.15 g |
| Glucose | 0.005 g |
| Boric acid | 0.6 g |
| Methylcellulose | 0.1 g |
| Sodium edetate | 0.01 g |
| Borax | q.s. |
| Polysorbate 60 | 0.3 g |
| 1-Menthol | 0.002 g |
| Chlorhexidine gluconate solution (20 W/V%) | 0.025 ml |
| Soybean ail | 0.3 g |
| Purified water | to 100 ml |
| pH | 7.0 |

According to the formula above, polysorbate 60 and methylcellulose were dissolved in water, and the mixture was warmed. To this, with vigorous stirring in a homo-mixer, was added soybean oil containing dissolved 1-menthol to form an emulsion. After cooling down to room temperature, to this were added sodium chloride, potassium chloride, glucose, boric acid, sodium edetate and the chlorhexidine gluconate solution, and then borax to adjust the pH, and the mixture was made to volume, which was filter-sterilized to give eye drops.

**[Preparation Example 4] Eye drops**

| | |
|---|---|
| Sodium chloride | 0.55 g |
| Potassium chloride | 0.15 g |
| Glucose | 0.005 g |
| Sodium chondroitin sulfate | 0.1 g |
| Boric acid | 0.4 g |
| Sodium citrate | 0.2 g |
| Hydroxypropylmethylcellulose 2910 | 0.1 g |
| Sodium edetate | 0.01 g |
| Borax | q.s. |
| Polyoxyethylenehydrogenated castor oil 60 | 2.0 g |
| 1-Menthol | 0.005 g |
| Sorbic acid | 0.1 g |
| Cottonseed oil | 1.0 g |
| Purified water | to 100 ml |
| pH | 6.0 |

According to the formula above, polyoxyethylenehydrogenated castor oil 60 and hydroxypropylmethylcellulose were dissolved in water, and the mixture was warmed. To this, with vigorous stirring in a homo-mixer, cottonseed oil containing dissolved 1-menthol was added to form an emulsion. After cooling down to room temperature, to this were added sodium chloride, potassium chloride, glucose, sodium chondroitin sulfate, boric acid, sodium citrate, sodium edetate and sorbic acid, and then borax to adjust the pH and the mixture was made to volume, which was filter-sterilized to give eye drops.

**[Preparation Example 5] Eye drops**

| | |
|---|---|
| Naphazoline hydrochloride | 0.005 g |
| Chlorpheniramine maleate | 0.03 g |
| Allantoin | 0.1 g |
| Pyridoxine hydrochloride | 0.1 g |
| Potassium L-aspartate | 0.5 g |
| Boric acid | 0.4 g |
| Sodium L-glutamate | 0.2 g |
| Decaglyceryl monolaureate | 5.0 g |
| 1-Menthol | 0.05 g |
| dl-Camphor | 0.01 g |
| Borneol . | 0.005 g |
| Sodium edetate | 0.01 g |
| Sorbic acid | 0.1 g |
| Sesame oil | 5.0 g |
| Hydrochloric acid | q.s. |
| Sodium hydroxide | q.s. |
| Purified water | to 100 ml |
| pH | 5.5 |

According to the formula above, decaglyceryl monolaureate was dissolved in water and the mixture was warmed. To this, with vigorous stirring in a homo-mixer, was added sesame oil containing dissolved 1-menthol, dl-camphor and borneol to form an emulsion. After cooling down to room temperature, to the were added naphazoline hydrochloride, chlorpheniramine maleate, allantoin, pyridoxine hydrochloride, potassium L-aspartate, boric acid, sodium L-glutamate, sodium edetate and sorbic acid, and then hydrochloric acid and sodium hydroxide to adjust the pH, and the mixture was made to volume, which was filter- sterilized to give eye drops.

**[Preparation Example 6] Eye drops**

| | |
|---|---|
| Sodium chloride | 0.55 g |
| Potassium chloride | 0.15 g |
| Potassium L-aspartate | 1.0 g |
| Sodium citrate | 0.2 g |
| Boric acid | 0.4 g |
| Soybean lecithin | 0.1 g |
| Sodium edetate | 0.01 g |
| Borax | q.s. |
| 1-Menthol | 0.005 g |
| Benzalkonium chloride | 0.005 g |
| Rapeseed oil | 0.1 g |
| Purified water . | to 100 ml |
| pH | 6.0 |

According to the formula above, soybean lecithin was dispersed in water and the mixture was warmed. To this, with vigorous stirring in a homo-mixer, was added rapeseed oil containing dissolved 1-menthol to form an emulsified. After cooling down to room temperature, to this were added sodium chloride, potassium chloride, potassium L-aspartate, boric acid, sodium citrate, sodium edetate and benzalkonium chloride, and then borax to adjust the pH, and the mixture was made to volume, which was filter-sterilized to give eye drops.

**[Preparation Example 7] Contact lens wetting liquid**

| | |
|---|---|
| Sodium chloride | 0.9 g |
| Polyvinyl alcohol | 2.0 g |
| Hydroxypropylmethyl cellulose 2906 | 0.5 g |
| Sodium acetate . | 0.1 g |
| Sodium edetate | 0.01 g |
| Polyoxyl stearate 40 | 0.2 g |
| 1-Menthol | 0.005 g |
| Benzalkonium chloride | 0.005 g |
| Camellia oil | 0.2 g |
| Hydrochloric acid | q.s. |
| Sodium hydroxide | q.s. |
| Purified water | to 100 ml |
| pH | 6.0 |

According to the formula above, polyvinyl alcohol was dissolved in warmed water, and hydroxypropylmethylcellulose then was dispersed in this and allowed to dissolve while cooling down. Polyoxyl stearate 40 then was added, and the mixture was warmed. To this, with vigorous stirring in a homo-mixer, was added camellia oil containing dissolved 1-menthol to form an emulsion. After cooling down to room temperature, to this were added sodium chloride, sodium acetate, sodium edetate and benzalkonium chloride, and then hydrochloric acid and sodium hydroxide to adjust the pH, and the mixture was made to volume, which was filter-sterilized to give a contact lens wetting liquid.

**[Preparation Example 8] Eye drops**

| | |
|---|---|
| Sodium chloride | 0.55 g |
| Potassium chloride | 0.15 g |
| Aminoethylsulfonic acid | 0.5 g |
| Boric acid | 0.6 g |
| Sodium edetate | 0.01 g |
| Borax | q.s. |
| Poloxamer 407 | 1 g |
| 1-Menthol | 0.002 g |
| Chlorhexidine gluconate solution (20 W/V%) | 0.025 ml |
| Migriol | 1 g |
| Purified water | to 100 ml |
| pH | 6.0 |

According to the formula above, water was added to poloxamer 407 to dissolve and the solution was warmed. To this, with vigorous stirring in a homo-mixer, was added Migriol containing dissolved 1-menthol to form an emulsion. After cooling down to room temperature, to this were added sodium chloride, potassium chloride, aminoethylsulfonic acid, boric acid, sodium edetate and the chlorhexidine gluconate solution, and then borax to adjust the pH, and the mixture was made to volume, which was filter-sterilized to give eye drops.

**[Preparation Example 9] Eye wash**

| | |
|---|---|
| Sodium chloride | 0.55 g |
| Potassium chloride | 0.15 g |
| Sodium chondroitin sulfate | 0.1 g |
| Boric acid | 0.6 g |
| Sodium edetate | 0.01 g |
| Borax | q.s. |
| Polyvinylpyrrolidone | 1.0 g |
| Lauryldimethylaminoacetic acid betaine | 0.3 g |
| Sorbic acid | 0.1 g |
| Corn oil | 0.3 g |
| Purified water | to 100 ml |
| pH | 6.0 |

According to the formula above, polyvinylpyrrolidone and lauryldimethylaminoacetic acid betaine were dissolved in water, and the solution was warmed. To this, with vigorous stirring in a homo-mixer, was added corn oil to form an emulsion. After cooling down to room temperature, to this were added sodium chloride, potassium chloride, sodium chondroitin sulfate, boric acid, sodium edetate and sorbic acid, and then borax to adjust the pH, and the mixture was made to volume, which then was filter-sterilized to give an eye wash.

**[Preparation Example 10] Eye drops**

| | |
|---|---|
| Sodium chloride | 0.55 g |
| Potassium chloride | 0.15 g |
| Aminoethylsulfonic acid | 0.5 g |
| Boric acid | 0.6 g |
| Sodium edetate | 0.01 g |
| Borax | q.s. |
| Sodium hyaluronate | 0.05 g |
| Sodium lauryl sulfate | 0.5 g |
| 1-Menthol | 0.002 g |
| Chlorhexidine gluconate solution (20 W/V%) | 0.025 ml |
| Olive oil | 1 g |
| Purified water | to 100 ml |
| pH | 6.0 |

According to the formula above, water was added to sodium lauryl sulfate and sodium hyaluronate to dissolve, and the solution was warmed. To this, with vigorous stirring in a homo-mixer, was added olive oil containing dissolved 1-menthol to form an emulsion. After cooling down to room temperature, to this were added sodium chloride, potassium chloride, aminoethylsulfonic acid, boric acid, sodium edetate and chlorhexidine gluconate solution, and then borax to adjust the pH, and the mixture was made to volume, which was filter-sterilized to give eye drops.

**[Preparation Example 11] Eye drops**

| | |
|---|---|
| Sodium chloride | 0.55 g |
| Potassium chloride | 0.15 g |
| Boric acid | 0.4 g |
| Sodium citrate | 0.2 g |
| Hydroxypropylmethylcellulose 2910 | 0.1 g |
| Sodium edetate | 0.01 g |
| Borax | q.s. |
| Polyoxyethylenehydrogenated castor oil 80 | 20 g |
| 1-Menthol | 0.005 g |
| Eucalyptus oil | 0.001 g |
| Sorbic acid | 0.1 g |
| Cottonseed oil | 20 g |
| Purified water | to 100 ml |
| pH | 6.0 |

According to the formula above, polyoxyethylenehydrogenated castor oil 80 was dissolved in warmed water, and hydroxypropylmethylcellulose were dispersed and allowed to dissolve while cooling down. Warmed again and with vigorous stirring in a homo-mixer, to this was added cottonseed oil containing dissolved 1-menthol and eucalyptus oil to form an emulsion. After cooling down to room temperature, to this were added sodium chloride, potassium chloride, boric acid, sodium citrate, sodium edetate and sorbic acid, and then borax to adjust the pH, and the mixture was made to volume, which was filter sterilized to give eye drops.

**[Preparation Example 12] Eye drops**

| | |
|---|---|
| Naphazoline hydrochloride | 0.005 g |
| Chlorpheniramine maleate | 0.03 g |
| Potassium L-aspartate | 0.5 g |
| Boric acid | 0.4 g |
| Sodium L-glutamate | 0.2 g |
| Polysorbate 40 | 10 g |
| 1-Menthol | 0.05 g |
| dl-Camphor | 0.01 g |
| Borneol | 0.005 g |
| Sodium edetate | 0.01 g |
| Sorbic acid | 0.1 g |
| Sesame oil | 5.0 g |
| Hydrochloric acid ... | q.s. |
| Sodium hydroxide | q.s. |
| Purified water | to 100 ml |
| pH | 5.5 |

According to the formula above, polysorbate 40 was dissolved in water and the solution was warmed. To this, with vigorous stirring in a homo-mixer, was added sesame oil containing dissolved 1-menthol, dl-camphor and borneol to form an emulsion. After cooling down to room temperature, to this were added naphazoline hydrochloride, chlorpheniramine maleate, potassium L-aspartate, boric acid, sodium L-glutamate, sodium edetate and sorbic acid, and then hydrochloric acid and sodium hydroxide to adjust the pH, and the mixture was made to volume, which was filter-sterilized to give eye drops.

**[Preparation Example 13] Eye drops**

| | |
|---|---|
| Chlorpheniramine maleate | 0.003 g |
| Dipotassium glycyrrhizinate | 0.025 g |
| Pyridoxine hydrochloride | 0.01 g |
| d-α-Tocopherol acetate | 0.005 g |
| Aminoethylsulfonic acid | 0.1 g |
| Boric acid | 1.6 g |
| Sodium edetate | 0.005 g |
| Borax . | q.s. |
| Polyoxyethylenehydrogenated castor oil 60 | 0.1 g |
| 1-Menthol | 0.002 g |
| Chlorobutanol | 0.1 g |
| Chlorhexidine gluconate solution (20 W/V%) | 0.025 ml |
| Castor oil | 1g |
| Purified water | to 100 ml |
| pH | 7.0 |

According to the formula above, water was added to polyoxyethylenehydrogenated castor oil 60 to dissolve and the solution was warmed.
To this, with vigorous stirring in a homo-mixer, was added castor oil containing dissolved 1-menthol and chlorobutanol, and an emulsion was formed. After cooling down to room temperature, to this were added chlorpheniramine maleate, dipotassium glycyrrhizinate, pyridoxine hydrochloride, d-α-tocopherol acetate, aminoethylsulfonic acid, boric acid, sodium edetate and chlorhexidine gluconate solution, and then borax to adjust the pH, and the mixture was made to volume, which was niter-sterilized to give eye drops.

**[Preparation Example 14] Eye drops**

| | |
|---|---|
| Sodium chloride | 0.55 g |
| Potassium chloride | 0.15 g |
| Potassium L-aspartate | 1.0 g |
| Sodium citrate | 0.2 g |
| Boric acid | 0.4 g |
| Polysorbate 80 | 15 g |
| Sodium edetate | 0.01 g |
| Borax | q.s. |
| 1-Menthol | 0.005 g |
| Borneol | 0.001 g |
| Geraniol | 0.001 g |
| Chlorobutanol | 0.01 g |
| Benzalkonium chloride | 0.005 g |
| Castor oil | 12 g |
| Purified water | to 100 ml |
| pH | 6.0 |

According to the formula above, water was added to polysorbate 80 to dissolve and the solution was warmed. To this, with vigorous stirring in a homo-mixer, was added castor oil containing dissolved 1-menthol, borneol, geraniol and chlorobutanol to form an emulsion. After cooling down to room temperature, to this were added sodium chloride, potassium chloride, potassium L-aspartate, boric acid, sodium citrate, sodium edetate and benzalkonium chloride, and then borax to adjust the pH, and the mixture was made to volume, which was filter-sterilized to give eye drops.

**[Preparation Example 15] Eye drops**

| | |
|---|---|
| Sodium chloride | 0.55 g |
| Potassium chloride | 0.15 g |
| Glucose | 0.005 g |
| Boric acid | 0.6 g |
| Methylcellulose | 0.1 g |
| Sodium edetate | 0.01 g |
| Borax | q.s. |
| Polysorbate 60 | 0.3 g |
| 1-Menthol | 0.002 g |
| Chlorobutanol | 0.01 g |
| Chlorhexidine gluconate solution (20 W/V%) | 0.025 ml |
| Soybean oil | 0.3 g |
| Purified water | to 100 ml |
| pH | 7.0 |

According to the formula above, polysorbate 60 and methylcellulose were dissolved in water, and the solution was warmed. To this, with vigorous stirring in a homo-mixer, was soybean oil containing dissolved 1-menthol and chlorobutanol to form an emulsion. After cooling down to room temperature, to this were added sodium chloride, potassium chloride, glucose, boric acid, sodium edetate and chlorhexidine gluconate solution, and then borax to adjust the pH, and the mixture was made to volume, which was filter-sterilized to give eye drops.

**[Preparation Example 16] Eye drops**

| | |
|---|---|
| Sodium chloride | 0.55 g |
| Potassium chloride | 0.15 g |
| Glucose | 0.005 g |
| Sodium chondroitin sulfate | 0.1 g |
| Boric acid | 0.4 g |
| Sodium citrate | 0.2 g |
| Hydroxypropylmethylcellulose 2910 | 0.1 g |
| Sodium edetate | 0.01 g |
| Borax | q.s. |
| Polyoxyethylenehydrogenated castor oil 60 | 2.0 g |
| 1-Menthol | 0.005 g |
| Chlorobutanol . | 0.05 g |
| Sorbic acid | 0.1 g |
| Cottonseed oil | 1.0 g |
| Purified water | to 100 ml |
| pH | 6.0 |

According to the formula above, polyoxyethylenehydrogenated castor oil 60 and hydroxypropylmethylcellulose were dissolved in water, and the solution was warmed. To this, with vigorous stirring in a homo-mixer, was added cottonseed oil containing dissolved 1-menthol and chlorobutanol to form an emulsion. After cooling down to room temperature, to this were added sodium chloride, potassium chloride, glucose, sodium chondroitin sulfate, boric acid, sodium citrate, sodium edetate and sorbic acid, and then borax to adjust the pH, and the mixture was made to volume, which was filter-sterilized to give eye drops.

**[Preparation Example 17] Eye drops**

| | |
|---|---|
| Naphazoline hydrochloride | 0.005 g |
| Chlorpheniramine maleate | 0.03 g |
| Allantoin . | 0.1 g |
| Pyridoxine hydrochloride | 0.1 g |
| Potassium L-aspartate | 0.5 g |
| Boric acid | 0.4 g |
| Sodium L-glutamate | 0.2 g |
| Decaglyceryl monolaureate . | 5.0 g |
| 1-Menthol | 0.05 g |
| dl-Camphor .. | 0.01 g |
| Borneol | 0.005 g |
| Sodium edetate | 0.01 g |
| Sorbic acid | 0.1 g |
| Chlorobutanol | 0.2 g |
| Sesame oil | 5.0 g |
| Hydrochloric acid | q.s. |
| Sodium hydroxide | q.s. |
| Purified water | to 100 ml |
| pH | 5.5 |

According to the formula above, decaglyceryl monolaureate was dissolved in water, and the solution was warmed. To this, with vigorous stirring in a homo-mixer, was added sesame oil containing dissolved 1-menthol, dl-camphor, borneol and chlorobutanol to form an emulsion. After cooling down to room temperature, to this were added naphazoline hydrochloride, chlorpheniramine maleate, allantoin, pyridoxine hydrochloride, potassium 1-aspartate, boric acid, sodium L-glutamate, sodium edetate and sorbic acid, and then hydrochloric acid and sodium hydroxide to adjust the pH, and the mixture was made to volume, which was filter-sterilized to give eye drops.

**[Preparation Example 18] Eye drops**

| | |
|---|---|
| Sodium chloride | 0.55 g |
| Potassium chloride | 0.15g |
| Potassium L-aspartate | 1.0 g |
| Sodium citrate | 0.2 g |
| Boric acid | 0.4 g |
| Soybean lecithin | 0.1 g |
| Sodium edetate | 0.01 g |
| Borax | q.s. |
| 1-Menthol | 0.005 g |
| Chlorobutanol | 0.01 g |
| Benzalkonium chloride | 0.005 g |
| Rapeseed oil | 0.1 g |
| Purified water | to 100 ml |
| pH | 6.0 |

According to the formula above, soybean lecithin was dispersed in water and the mixture was warmed. To this, with vigorous stirring in a homo-mixer, was added rapeseed oil containing dissolved 1-menthol and chlorobutanol to form an emulsion. After cooling down to room temperature, to this were added sodium chloride, potassium chloride, potassium L-aspartate, boric acid, sodium citrate, sodium edetate and benzalkonium chloride, and then borax to adjust the pH, and the mixture was made to volume, which was filter-sterilized to give eye drops.

**[Preparation Example 19] Contact lens wetting liquid**

| | |
|---|---|
| Sodium chloride | 0.9 g |
| Polyvinyl alcohol | 2.0 g |
| Hydroxypropylmethyl cellulose 2906 | 0.5 g |
| Sodium acetate | 0.1 g |
| Sodium edetate | 0.01 g |
| Polyoxyl stearate 40 | 0.2 g |
| 1-Menthol | 0.005 g |
| Chlorobutanol | 0.05 g |
| Benzalkonium chloride | 0.005 g |
| Camellia oil | 0.2 g |
| Hydrochloric acid | q.s. |
| Sodium hydroxide | q.s. |
| Purified water | to 100 ml |
| pH | 6.0 |

According to the formula above, polyvinylalcohol was dissolved in warmed water, and hydroxypropylmethylcellulose was dispersed and allowed to dissolve while cooling down. Polyoxyl stearate 40 was added, and the mixture was warmed. To this, with vigorous stirring in a homo-mixer, was added camellia oil containing dissolved 1-menthol and chlorobutanol to form an emulsion. After cooling down to room temperature, to this were added sodium chloride, sodium acetate, sodium edetate and benzalkonium chloride, and then hydrochloric acid and sodium hydroxide to adjust the pH, and the mixture was made to volume, which was filter-sterilized to give a contact lens wetting liquid.

**[Preparation Example 20] Eye drops**

| | |
|---|---|
| Sodium chloride | 0.55 g |
| Potassium chloride . | 0.15 g |
| Sodium chondroitin sulfate | 0.1 g |
| Boric acid . | 0.6 g |
| Sodium edetate | 0.01 g |
| Borax | q.s. |
| Egg yolk lecithin | 0.5 g |
| 1-MenthoI | 0.01 g |
| Chlorobutanol | 0.05 g |
| Chlorhexidine gluconate solution (20 W/V%) | 0.025 ml |
| Purified water | to 100 ml |
| pH | 6.0 |

According to the formula above, egg yolk lecithin was dispersed in water and the mixture was warmed. To this, with vigorous stirring, 1-menthol and chlorobutanolwere added to dissolve to form an emulsion. After cooling down to room temperature, to this were added sodium chloride, potassium chloride, sodium chondroitin sulfate, boric acid, sodium edetate and chlorhexidine gluconate solution (20 W/V%), and then borax to adjust the pH, and the mixture was made to volume, which was filter-sterelized to give eye drops.

**[Preparation Example 21] Eye drops**

| | |
|---|---|
| Sodium chloride | 0.55 g |
| Potassium chloride | 0.15 g |
| Aminoethylsulfonic acid | 0.5 g |
| Boric acid | 0.6 g |
| Sodium edetate | 0.01 g |
| Borax. | q.s. |
| Poloxamer 407 | 1 g |
| 1-Menthol | 0.002 g |
| Chlorobutanol | 0.1 g |
| Chlorhexidine gluconate solution (20 W/V%) | 0.025 ml |
| Migriol | 1 g |
| Purified water | to 100 ml |
| pH | 6.0 |

According to the formula above, water was added to poloxamer 407 to dissolve. and the solution was warmed. To this, with vigorous stirring in a homo-mixer, was added Migriol containing 1-menthol and chlorobutanol to form an emulsion. After cooling down to room temperature, to this were added sodium chloride, potassium chloride, aminoethylsulfonic acid, boric acid, sodium edetate and chlorhexidine gluconate solution, and then borax to adjust the pH, and the mixture was made to volume, which was filter-sterilized to form eye drops.

**[Preparation Example 22] Eye wash**

| | |
|---|---|
| Sodium chloride | 0.55 g |
| Potassium chloride | 0.15 g |
| Sodium chondroitin sulfate | 0.1 g |
| Boric acid | 0.6 g |
| Sodium edetate | 0.01 g |
| Borax | q.s. |
| Polyvinylpyrrolidone | 1.0 g |
| Lauryldimethylaminoacetic acid betaine | 0.3 g |
| Chlorobutanol | 0.05 g |
| Sorbic acid | 0.1g |
| Corn oil | 0.3g |
| water Purified water | ml to 100 ml |
| pH | 6.0 |

According to the formula above, polyvinylpyrrolidone and lauryldimethylaminoacetic acid betaine were dissolved in water, and the solution was warmed. To this, with vigorous stirring in a homo-mixer, was added corn oil containing dissolved chlorobutanol to form an emulsion. After cooling down to room temperature, to this were added sodium chloride, potassium chloride, sodium chondroitin sulfate, boric acid, sodium borate and sorbic acid, and then borax to adjust the pH, and the mixture was made to volume, which was then filter-sterilized to give an eye wash.

**[Preparation Example 23] Eye drops**

| | |
|---|---|
| Sodium chloride | 0.55 g |
| Potassium chloride | 0.15 g |
| Aminoethylsulfonic acid | 0.5 g |
| Boric acid | 0.6 g |
| Sodium edetate | 0.01 g |
| Borax | q.s. |
| Sodium hyaluronate | 0.05 g |
| Sodium lauryl sulfate | 0.5 g |
| 1-Menthol | 0.002 g |
| Chlorobutanol | 0.1 g |
| Chlorhexidine gluconate solution (20 W/V%) | 0.425 ml |
| Olive oil | 1 g |
| Purified water | to 100 ml |
| pH | 6.0 |

According to the formula above, sodium lauryl sulfate and sodium hyaluronate were added to water to dissolve, and the solution was warmed. To this, with vigorous stirring in a homo-mixer, was added olive oil containing dissolved 1-menthol and chlorobutanol to form an emulsion. After cooling down to room temperature, to this were added sodium chloride, potassium chloride, aminoethylsulfonic acid, boric acid, sodium edetate and chlorhexidine gluconate solution, and then borax to adjust the pH, the mixture was made to volume, which then was filter-sterilized to give eye drops.

**[Preparation Example 24] Eye drops**

| | |
|---|---|
| Sodium chloride | 0.55 g |
| Potassium chloride | 0.15 g |
| Boric acid | 0.4 g |
| Sodium citrate | 0.2 g |
| Hydroxypropylmethylcellulose 2910 | 0.1 g |
| Sodium edetate | 0.01 g |
| Borax | q.s. |
| Polyoxyethylenehydrogenated castor oil 80 | 20 g |
| 1-Menthol | 0.005 g |
| Eucalyptus oil | 0.001 g |
| Chlorobutanol | 0.05 g |
| Sorbic acid | 0.1 g |
| Cottonseed oil | 20 g |
| Purified water | to 100 ml |
| pH | 6.0 |

According to the formula above, polyoxyethylenehydrogenated castor oil 80 was dissolved in warmed water, and in this was dispersed hydroxypropylmethylcellulose, and allowed to dissolve while cooling down. The mixture was warmed again, and to this, with vigorous stirring in a homo-mixer, was added cottonseed oil containing 1-menthol, eucalyptus oil and chlorobutanol to form an emulsion. After cooling down to room temperature, to this were sodium chloride, potassium chloride, boric acid, sodium citrate, sodium edetate and sorbic acid, and then borax to adjust the pH, and the mixture was made to volume, which then was filter-sterilized to give eye drops.

**[Preparation Example 25] Eye drops**

| | |
|---|---|
| Naphazoline hydrochloride | 0.005 g |
| Chlorpheniramine maleate | 0.03 g |
| Potassium L-aspartate | 0.5 g |
| Boric acid | 0.4 g |
| Sodium L-glutamate | 0.2 g |
| Polysorbate 40 | 10 g |
| 1-Menthol | 0.05 g |
| dl-Camphor | 0.01 g |
| Borneol | 0.005 g |
| Sodium edetate | 0.01 g |
| Sorbic acid | 0.1 g |
| Chlorobutanol | 0.2 g |
| Sesame oil | 5.0 g |
| Hydrochloric acid | q.s. |
| Sodium hydroxide | q.s. |
| Purified water | to 100 ml |
| pH | 5.5 |

According to the formula above, polysorbate 40 was dissolved in water and the solution was warmed. To this, with vigorous stirring in a homo-mixer, was added sesame oil containing dissolved 1-menthol, dl-camphor, borneol and chlorobutanol and to form an emulsion. After cooling down to room temperature, to this were added naphazoline hydrochloride, chlorpheniramine maleate, potassium L-aspartate, boric acid, sodium L-glutamate, sodium edetate and sorbic acid, and then hydrochloric acid and sodium hydroxide to adjust the pH, and the mixture was made to volume, which then was filter-sterilized to give eye drops.

**[Preparation Example 26] Eye drops**

| | |
|---|---|
| Chlorpheniramine maleate | 0.02 g |
| Tetrahydrozoline hydrochloride | 0.05 g |
| Panthenol | 0.1 g |
| ε-aminocaproic acid | 1.0 g |
| Sodium chloride | 0.3 g |
| Boric acid | 0.5 g |
| Borax | q.s. |
| Sodium edetate | 0.01 g |
| Polysorbate 80 | 0.5 g |
| Castor oil | 0.5 g |
| Chlorobutanol | 0.1 g |
| Peppermint oil | 0.01 g |
| Sorbic acid | 0.2 g |
| Purified water | to 100 ml |
| pH | 6.0 |

According to the formula above, water was added to polysorbate 80 to dissolve, and the solution was warmed. To this, with vigorous stirring in a homo-mixer, was added castor oil containing dissolved peppermint oil and chlorobutanol to form an emulsion. After cooling down to room temperature, to this were added chlorpheniramine maleate, tetrahydrozoline hydrochloride, panthenol, ε-aminocaproic acid, sodium chloride, boric acid, sodium edetate and sorbic acid, and then borax to adjust pH, and the mixture was made to volume, which then was filter-sterilized to give eye drops.

**[Preparation Example 27] Eye drops**

| | |
|---|---|
| Chlorpheniramine maleate | 0.03 g |
| Tetrahydrozoline hydrochloride | 0.05 g |
| Calcium pantothenate | 0.1 g |
| Allantoin | 0.1 g |
| Sodium chloride | 0.5 g |
| Sodium citrate | 0.4 g |
| Boric acid | 0.2 g |
| Sodium edetate | 0.01 g |
| Polysorbate 80 | 3 g |
| Castor oil | 2 g |
| Chlorobutanol | 0.2 g |
| 1-Menthol | 0.02 g |
| Bergamot oil | 0.001 g |
| Hydrochloric acid | q.s. |
| Sodium hydroxide | q.s. |
| Chlorhexidine gluconate solution (20 W/V%) | 0.025 ml |
| Purified water | to 100 ml |
| pH | 5.5 |

According to the formula above, water was added to polysorbate 80 to dissolve, and the mixture was warmed. To this, with vigorous stirring in a homo-mixer, was added castor oil containing dissolved 1-menthol, bergamot oil and chlorobutanol to form an emulsion. After cooling down to room temperature, to this were added chlorpheniramine maleate, tetrahydrozoline hydrochloride, calcium pantothenate, allantoin, sodium chloride, sodium citrate, boric acid, sodium edetate and chlorhexidine gluconate solution, and then hydrochloric acid and sodium hydroxide to adjust the pH, and the mixture was made to volume, which then was filter-sterilized to give eye drops.

### INDUSTRIAL APPLICABILITY

According to the present invention, adsorption of refreshing agents and chlorobutanol by contact lenses is markedly suppressed, and the pH decline in the neutral range is significantly suppressed in spite of the presence of chlorobutanol. Thus, the present invention can be applied in the production of ophthalmic compositions for contact lens, which contain one or more refreshing agents or chlorobutanol, or both in combination, at higher concentrations than before.

## Claims

1. An ophthalmic composition for contact lens, wherein the composition is an oil-in-water type emulsion comprising a refreshing agent and/or chlorobutanol together with an oil and an emulsifying agent in water.

2. The ophthalmic composition for contact lens of claim 1, wherein the refreshing agent is at least one compound selected from the group consisting of terpenoids, clove perfume, peppermint perfume, eucalyptus perfume, lemon perfume, laurel perfume, lavender perfume, tarragon perfume, cardamon perfume, cedar perfume, grapefruit perfume, orange perfume, ginger perfume, bergamot perfume, coriander perfume, cinnamon perfume, jasmine perfume, rosemary perfume, rose perfume, pine perfume, cardamon perfume, benzoin perfume, geranium perfume, chamomile perfume, marjoram perfume, spearmint perfume, fennel perfume, vanilla flavor, peppermint oil, peppermint water and bergamot oil.

3. The ophthalmic composition for contact lens of claim 2, wherein the terpenoid is at least one compound selected from the group consisting of menthol, camphor, borneol, geraniol, menthone, cineol and limonene.

4. The ophthalmic composition for contact lens of one of claims 1 to 3,
wherein the oil is an animal/vegetable oil.

5. The ophthalmic composition for contact lens of claim 4, wherein the vegetable/animal oil is castor oil.

6. The ophthalmic composition for contact lens of one of claims 1 to 5,
wherein the emulsifying agent is at least one compound selected from the group consisting of surfactants, phospholipids and water-soluble macromolecular compounds.

7. The ophthalmic composition for contact lens of claim 6, wherein the surfactants are nonionic surfactants.

8. The ophthalmic composition for contact lens of claim 7, wherein the nonionic surfactants are polyoxyethylenesorbitan fatty acid esters.

9. The ophthalmic composition for contact lens of one of claims 6 to 8,
wherein the phospholipid is lecithin.

10. The ophthalmic composition for contact lens of one of claims 6 to 9,
wherein the water-soluble macromolecular compound is hydroxypropylmethylcellulose.

11. The ophthalmic composition for contact lens of one of claims 1 to 10,
wherein the contact lens is a soft contact lens.

12. An ophthalmic composition for contact lens, wherein the composition is an oil-in-water type emulsion containing chlorobutanol and a phospholipid in water.

13. The ophthalmic composition for contact lens of claim 12 or 13 further containing a refreshing agent.

14. The ophthalmic composition for contact lens of claim 13, wherein the refreshing agent is at least one compound selected from the group consisting of terpenoids, clove perfume, peppermint perfume, eucalyptus perfume, lemon perfume, laurel perfume, lavender perfume, tarragon perfume, cardamon perfume, cedar perfume, grapefruit perfume, orange perfume, ginger perfume, bergamot perfume, coriander perfume, cinnamon perfume, jasmine perfume, rosemary perfume, rose perfume, pine perfume, cardamon perfume, benzoin perfume, geranium perfume, chamomile perfume, marjoram perfume, spearmint perfume, fennel perfume, vanilla flavor, peppermint oil, peppermint water and bergamot oil.

15. A method for suppressing adsorption of a refreshing agent and/or chlorobutanol by a contact lens in an aqueous ophthalmic composition for contact lens containing the refreshing agent and/or chlorobutanol, wherein the method comprises preparing the composition in the form of an oil-in-water type emulsion by adding an oil and an emulsifying agent.

16. The method for suppressing adsorption of claim 15, wherein the oil is an animal/vegetable oil.

17. The method for suppressing adsorption of claim 16, wherein the animal/vegetable oil is castor oil.

18. The method for suppressing adsorption of one of claim 15 to 17,
wherein the emulsifying agent is at least one compound selected from the group consisting of surfactants, phospholipids and water-soluble macromolecular compounds.

19. The method for suppressing adsorption of claim 18, wherein the surfactants are nonionic surfactants.

20. The method for suppressing adsorption of claim 19, wherein the nonionic surfactants are polyoxyethylenesorbitan fatty acid esters.

21. The method for suppressing adsorption of one of claims 18 to 20,
wherein the phospholipid is lecithin.

22. The method for suppressing adsorption of one of claims 18 to 20,
wherein the water-soluble macromolecular compound is hydroxypropylmethylcellulose.

23. A method for suppressing adsorption of chlorobutanol by a contact lens in a chlorobutanol-containing aqueous ophthalmic composition for contact lens, wherein the method comprises preparing the composition in the form of an oil-in-water type emulsion by adding a phospholipid.

24. A method for suppressing pH decline of a chlorobutanol-containing aqueous ophthalmic composition for contact lens, wherein the method comprises preparing the composition in the form of oil-in-water type emulsion by adding an oil and an emulsifying agent.

25. The method for suppressing pH decline of claim 24, wherein the oil is an animal/vegetable oil.

26. The method for suppressing pH decline of claim 25, wherein the animal/vegetable oil is castor oil.

27. The method for suppressing pH decline of one of claim 24 to 26,
wherein the emulsifying agent is at least one compound selected from the group consisting of surfactants, phospholipids and water-soluble macromolecular compounds.

28. The method for suppressing pH decline of claim 27 above, wherein the surfactants are nonionic surfactants.

29. A method for suppressing pH decline of a chlorobutanol-containing aqueous ophthalmic composition for contact lens, wherein the method comprises preparing the composition in the form of oil-in-water type emulsion by adding a phospholipid.
